# EUROPÄISCHE PATENTSCHRIFT

(11) **EP 1 054 636 B1**
(45) Veröffentlichungstag und Bekanntmachung des Hinweises auf die Patenterteilung: **31.10.2001**
(21) Anmeldenummer: 00909288.3
(22) Anmeldetag: 02.03.2000
(51) Int. Cl.: A61B 17/28

(54) **HANDGRIFF FÜR EIN MEDIZINISCHES INSTRUMENT**
HANDLE FOR A MEDICAL INSTRUMENT
POIGNEE POUR INSTRUMENT MEDICAL

(30) Priorität: 17.03.1999 DE 19912038
(43) Veröffentlichungstag der Anmeldung: 29.11.2000
(73) Patentinhaber: Karl Storz GmbH & Co. KG, 78532 Tuttlingen (DE)
(72) Erfinder: FRANK, Timothy, Graham, Dundee DD1 9SY (GB); BACHER, Uwe, D-78532 Tuttlingen (DE); CUSCHIERI, Alfred, Dundee DD1 9SY (GB)
(74) Vertreter: Heuckeroth, Volker
(86) Internationale Anmeldenummer: EP0001788
(87) Internationale Veröffentlichungsnummer: WO0054674

(56) Entgegenhaltungen:
- DE-A- 4 318 019
- DE-A- 4 431 561
- DE-A- 19 632 135

## Beschreibung

Die Erfindung betrifft einen Handgriff für ein medizinisches Rohrschaftinstrument, mit zumindest einem beweglichen Griffteil und mit einem Kopplungsteil, durch das ein Rohrschaft mit dem Handgriff verbindbar ist, wobei das bewegliche Griffteil mit einem in Richtung einer Längsachse des Rohrschafts axial beweglichen Kraftübertragungselement verbindbar ist, wobei eine Bewegung des zumindest einen beweglichen Griffteils in eine axiale Bewegung des Kraftbertragungselements übertragen wird, und wobei das Kopplungsteil um eine quer zur Längsachse des Rohrschafts verlaufende Drehachse relativ zu einer Handgriffachse verschwenkbar und arretierbar ist.

Ein derartiger Handgriff ist aus der DE 196 32 135 Al bekannt.

Rohrschaftinstrumente werden für unterschiedlichste Arten von operativen Eingriffen am menschlichen oder tierischen Körper, insbesondere in der minimalinvasiven Chirurgie, verwendet. Solche Rohrschaftinstrumente können als Zangen, bspw. Präparier- und Faßzangen, Zangen zum Abtrennen bzw. Schneiden von Gewebe, als Stanzen oder dergleichen ausgebildet sein, wobei sich die Rohrschaftinstrumente hinsichtlich ihrer unterschiedlichen Funktion durch eine entsprechende Ausgestaltung ihrer Werkzeuge am distalen Ende unterscheiden. Als Werkzeuge am distalen Ende des Rohrschafts können Schneid-, Faß- oder Stanzwerkzeuge vorgesehen sein, wobei ein Rohrschaftinstrument zumindest ein bewegliches Werkzeug, beispielsweise in Form eines Maulteils, aufweist. Ein solches Rohrschaftinstrument kann jedoch am distalen Ende auch zwei bewegliche Werkzeuge aufweisen.

Zur Betätigung des zumindest einen beweglichen Werkzeugs weisen Rohrschaftinstrumente am proximalen Ende des Rohrschafts einen Handgriff auf. Der Rohrschaft des Rohrschaftinstruments ist üblicherweise mit einem Kopplungsteil des Handgriffs unlösbar oder lösbar verbunden. Weiterhin weist der Handgriff zumindest ein bewegliches Griffteil auf, das der eigentlichen Betätigung des zumindest einen beweglichen Werkzeugs am distalen Ende des Rohrschafts dient. Zur Betätigung des zumindest einen beweglichen Werkzeugs am distalen Ende des Rohrschafts ist das zumindest eine bewegliche Griffteil des Handgriffs über ein in Richtung der Längsachse des Rohrschafts axial bewegliches Kraftübertragungselement, beispielsweise eine Zug- oder Schubstange, mit dem zumindest einen beweglichen Werkzeug kraftschlüssig verbunden, so daß eine Bewegung des beweglichen Griffteils, bspw. eine Schwenk- oder Axialbewegung, in eine axiale Relativbewegung des Kraftübertragungselements bezüglich des Rohrschafts und schließlich in eine Bewegung des beweglichen Werkzeugs übertragen wird.

Ein Handgriff für ein Rohrschaftinstrument ist in unterschiedlichen Ausgestaltungen bekannt. In der Regel weist der Handgriff ein zweites Griffteil auf, das entweder unbeweglich und somit schaftfest oder ebenfalls beweglich ist. Ein solcher Handgriff kann weiterhin in der Art eines Scherengriffs ausgebildet sein, bei dem die beiden Griffteile seitlich von dem Rohrschaft des Rohrschaftinstruments abstehen. Bei dieser Art von Handgriffen ist das zumindest eine bewegliche Griffteil um eine quer zur Längsachse des Rohrschafts verlaufende Drehachse gelenkig mit dem anderen Griffteil verbunden. Im Sinne der vorliegenden Erfindung kann der Handgriff jedoch auch in der Art eines Pistolengriffes oder auch so ausgebildet sein, daß der Handgriff als Stabgriff in der Faust einer Hand gehalten wird.

All diesen bekannten Arten von Handgriffen ist gemeinsam, daß die Handgriffe im mit dem Rohrschaft verbundenen Zustand bezüglich dem Rohrschaft eine unveränderliche Winkelstellung einnehmen, d.h. daß eine Handgriffachse und die Längsachse des Rohrschafts einen festen Winkel miteinander bilden.

Ein Handgriff, der bezüglich der Längsachse des Schafts eine unveränderliche Winkellage einnimmt, ist jedoch hinsichtlich seiner Handhabungseigenschaften nicht immer an die Anforderungen des das Instrument bedienenden Arztes angepaßt. Je nach Gewohnheit werden von unterschiedlichen Ärzten unterschiedliche Griffstellungen desselben Handgriffstyps bezüglich des Schafts bevorzugt. Um stets den Handgriff mit bezüglich dem Schaft optimal ergonomischer Winkelstellung zur Verfügung zu haben, wäre es daher erforderlich, für jede Art von Handgriffen einen Satz mehrerer dieser Handgriffe bereitzuhalten, die dann jeweils im mit dem Schaft verbundenen Zustand eine andere Winkellage zu dem Schaft einnehmen, so daß jeder Arzt den für ihn ergonomisch optimalen Handgriff aussuchen kann. Im Fall, daß die Handgriffe nicht auswechselbar sind, bedeutet dies, daß sogar von jedem Instrument ein ganzer Satz solcher Instrumente mit unterschiedlich abgewinkelten Handgriffen bereitgehalten werden muß.

Zusätzlich kann es im Einzelfall auch wünschenswert oder erforderlich sein, daß der Handgriff bei ein und demselben Instrument während eines Operationsvorgangs unterschiedliche Winkelstellungen bezüglich des Schafts einnehmen kann, um bei einem operativen Eingriff die jeweils bequemste und damit für den gerade durchzuführenden Operationsvorgang sicherste Handstellung zu haben. Bei den bekannten Handgriffen, von denen jeweils ein ganzer Satz vorhanden wäre, würde dies bedeuten, daß während des Operationsvorgangs mehrmals der Handgriff ausgetauscht oder bei nicht abnehmbaren Handgriffen sogar das ganze Instrument ausgetauscht werden müßte, was die Operationsdauer wesentlich verlängern würde.

Die bekannten Handgriffe erweisen sich demnach hinsichtlich ihrer ergonomischen Eigenschaften als nachteilig.

Demgegenüber weist das aus der eingangs genannten DE 196 32 135 Al bekannte Instrument einen abwinkelbaren Handgriff auf, so daß die ergonomischen Eigenschaften dieses bekannten Handgriffs bzw. des damit ausgestatteten Instruments gegenüber den zuvor genannten bekannten Handgriffen und Instrumenten verbessert ist.

Die Übertragung der Bewegung des beweglichen Griffteils auf das Kraftübertragungselement im Rohrschaft erfolgt bei diesem bekannten Handgriff durch eine an das bewegliche Griffteil angeschweißte Exzenterscheibe. Die Exzenterscheibe wirkt unter Reibung mit einem Stößel zusammen, wodurch einerseits die Kraftübertragung wegen der aneinander unter Gleitreibung vorbei geführten Teile einem Verschleiß unterliegt, und andererseits zwischen der Exzenterscheibe und dem Stößel Reibungsverluste in der Kraftübertragung auftreten können.

Der Erfindung liegt daher die Aufgabe zugrunde, für die Kraftübertragung vom beweglichen Griffteil auf das Kraftübertragungselement bei einem verschwenkbaren Handgriff einen alternativen Mechanismus anzugeben.

Hinsichtlich des eingangs genannten Handgriffs wird diese Aufgabe erfindungsgemäß dadurch gelöst, daß das Kopplungsteil um zumindest eine erste quer zur Längsachse des Schafts verlaufende Drehachse relativ zu einer Handgriffachse verschwenkbar ist, daß das bewegliche Griffteil mit dem Kraftübertragungselement über eine Hebelanordnung verbindbar ist, die zumindest zwei zweiarmige Hebel aufweist, von denen ein erster Hebel um eine handgriffeste Achse und ein zweiter Hebel um eine kopplungsteilfeste Achse schwenkbar ist, und daß die beiden Hebel kraftschlüssig und um die erste Drehachse gegeneinander verdrehbar miteinander verbunden sind.

Erfindungsgemäß ist demnach zunächst vorgesehen, das Kopplungsteil, über das der Handgriff mit dem Schaft verbindbar ist, verschwenkbar auszugestalten, so daß sich der Handgriff bezüglich des Schafts in verschiedenen Winkelstellungen abwinkeln läßt. In der eingestellten Winkelstellung des Handgriffs läßt sich dieser dann bevorzugt arretieren, um den Schaft starr mit dem Handgriff zu verbinden, um das Instrument dann für einen Operationsvorgang zu verwenden. Durch die verschwenkbare Ausgestaltung des Kopplungsteils kann der Arzt jeweils die für ihn optimale ergonomische Winkelstellung zwischen dem Handgriff und dem Schaft selbst einstellen, ohne den Handgriff oder gar das ganze Instrument auswechseln zu müssen. Hinsichtlich der Ergonomie ist der erfindungsgemäße Handgriff somit wesentlich verbessert.

Durch die Verstellbarkeit der Handgriffachse bezüglich der Längsachse des Schafts ergibt sich das Problem, das Kraftübertragungselement so mit dem beweglichen Griffteil zu verbinden, daß in jeder Winkelstellung des Handgriffs die Bewegung des beweglichen Griffteils in eine axiale Bewegung des Kraftübertragungselements übertragen werden kann. Bei den herkömmlichen Handgriffen ist das Kraftübertragungselement, das üblicherweise als Zug- oder Schubstange ausgebildet ist, mit seinem proximalen Ende direkt mit dem beweglichen Griffteil verbunden. Diese Art der Verbindung des Kraftübertragungselements läßt sich jedoch nicht aufrechterhalten, wenn der Handgriff gegenüber dem Schaft abwinkelbar ist. Dies würde nämlich bedeuten, daß das Kraftübertragungselement im Bereich der Drehachse des Kopplungsteils ebenfalls umgelenkt, abgewinkelt oder abgeknickt werden müßte, wobei zusätzlich je nach Winkelstellung des Schafts zur Handgriffachse ein Längenausgleich für das kraftübertragungselement vorgesehen werden müßte. Die Verwendung eines flexiblen Kraftübertragungselements, beispielsweise in Form eines Bowdenzuges, das sich an unterschiedliche Winkelstellungen im abgewinkelten Bereich anpassen könnte, hätte jedoch den Nachteil, daß im Bereich der Umlenkungsstelle eine erhöhte Reibung auftreten würde, und daß sich beim Abwinkeln des Schafts bezüglich der Handgriffachse wegen der konstanten Länge des Kraftübertragungselements das bewegliche Griffteil unerwünscht bewegen würde. Ein Bowdenzug hat weiterhin den Nachteil, daß sich zwischen dem Mantel und der flexiblen Seele des Bowdenzugs Verunreinigungen ansammeln können, die nicht oder nur erschwert entfernbar sind. Des weiteren lassen sich mit einem Bowdenzug nur beschränkt Schubkräfte auf das bewegliche Werkzeug übertragen.

Demgegenüber ist erfindungsgemäß eine Kraftübertragungsmechanik zwischen dem beweglichen Griffteil und dem Kraftübertragungselement vorgesehen, bei der das bewegliche Griffteil mit dem Kraftübertragungselement über eine Hebelanordnung verbindbar ist, die zumindest zwei zweiarmige Hebel aufweist, von denen ein erster Hebel um eine handgriffeste Achse und ein zweiter Hebel um eine kopplungsteilfeste Achse schwenkbar ist, und bei der die beiden Hebel kraftschlüssig und um die erste Drehachse gegeneinander verdrehbar miteinander verbunden sind.

Anstatt also das Kraftübertragungselement über die erste Drehachse hinweg direkt mit dem beweglichen Griffteil zu verbinden, ist bei dem erfindungsgemäßen Handgriff zwischen das bewegliche Griffteil und das Kraftübertragungselement eine Hebelanordnung aus zumindest zwei zweiarmigen Hebeln geschaltet.

Der Kraftfluß wird zunächst von dem beweglichen Griffteil in den ersten Hebel eingeleitet, so daß sich dieser um seine handgriffeste Achse verschwenkt und über die kraftschlüssige Verbindung mit dem zweiten Hebel diesen um seine kopplungsteilfeste Achse verschwenkt, der wiederum mit dem Kraftübertragungselement in Verbindung steht, so daß dieses dann axial in Richtung des Schafts bewegt wird. Dadurch, daß die beiden Hebel um die erste Drehachse gegeneinander verdrehbar miteinander verbunden sind, können die beiden Hebel relativ zueinander jeweils eine Winkelstellung einnehmen, die der jeweils eingestellten Winkelstellung des Schafts bezüglich der Handgriffachse entspricht. In jeder Winkelstellung liegen somit die gleichen Kraftübertragungsverhältnisse vor, da das Kraftübertragungsverhältnis lediglich durch die Ausgestaltung der beiden Hebel bestimmt wird. Insbesondere ist bei dieser Ausgestaltung der Kraftübertragungsmechanik von Vorteil, daß die Kraftübertragung von dem beweglichen Griffteil auf das Kraftübertragungselement unabhängig von der Winkelverstellbarkeit des Schafts bezüglich der Handgriffachse ist. Ein weiterer Vorteil dieser Ausgestaltung besteht darin, daß beim Betätigen des beweglichen Griffteils und damit bei einem Kraftfluß durch die Hebelanordnung der Schaft bezüglich der Handgriffachse verschwenkt werden kann, ohne das bewegliche Griffteil dazu loslassen oder inaktivieren zu müssen.

Somit wird die der Erfindung zugrunde liegende Aufgabe vollkommen gelöst.

In einer bevorzugten Ausgestaltung ist das bewegliche Griffteil mit einer in ihrer Längsrichtung axial verschiebbaren Koppelstange verbunden, die mit einem ersten Hebelarm des ersten Hebels verbunden ist.

Die Krafteinleitung vom beweglichen Griffteil in die Hebelanordnung über eine axial verschiebbaren Koppelstange hat den Vorteil, daß über die Koppelstange eine Krafteinleitung von dem beweglichen Griffteil ermöglicht wird, auch wenn das Griffteil von der Hebelanordnung entfernt angeordnet ist.

In einer weiteren bevorzugten Ausgestaltung ist die Koppelstange so angeordnet, daß ihre Längsachse auf einer Geraden liegt, die die erste Drehachse schneidet.

Hierbei ist von besonderem Vorteil, daß die Koppelstange beim Betätigen des beweglichen Griffteils kein Drehmoment auf das Kopplungsteil ausübt, das ja um die erste Drehachse verschwenkbar ist. Mit anderen Worten wird ein Spiel des Kopplungsteils um die erste Drehachse nicht auf das bewegliche Griffteil übertragen, so daß eine nahezu spielfreie Kraftübertragungsmechanik von dem beweglichen Griffteil auf das Kraftübertragungselement erreicht wird.

In einer weiteren bevorzugten Ausgestaltung sind der erste Hebel und der zweite Hebel durch einen Zapfen miteinander verbunden, der ein Drehgelenk aufweist, dessen Drehachse mit der ersten Drehachse zusammenfällt.

Durch diese Maßnahme wird eine konstruktiv vorteilhaft besonders einfache um die erste Drehachse drehbare Verbindung zwischen dem ersten Hebel und dem zweiten Hebel geschaffen. Der Zapfen kann dabei so angeordnet sein, daß seine Längsachse mit der ersten Drehachse zusammenfällt. Der Zapfen kann im einfachsten Fall aus zwei Zapfenhälften bestehen, die über ein Gleitgelenk miteinander verbunden sind, das einen Rotationsfreiheitsgrad um die erste Drehachse aufweist.

Dabei ist es weiter bevorzugt, wenn der erste Hebel eine axiale Bewegung der Koppelstange in eine axiale Bewegung des Zapfens in Richtung der ersten Drehachse umsetzt, und der zweite Hebel die axiale Bewegung des Zapfens in eine axiale Bewegung des Kraftübertragungselements in Richtung der Längsachse des Schafts umsetzt.

Durch diese Maßnahme wird eine kinematisch besonders günstige Kraftübertragung von der Koppelstange auf das Kraftübertragungselement durch die Hebelanordnung hindurch ermöglicht. Da die Hebelanordnung zumindest zwei Hebel aufweist, kann die Hebelanordnung zusammen mit dem Zapfen günstigerweise so ausgebildet sein, daß die axiale Bewegung der Koppelstange von dem ersten Hebel in eine axiale Bewegung des Zapfens quer zur Bewegungsrichtung der Koppelstange umgesetzt wird, wobei dann die axiale Bewegung des Zapfens durch den zweiten Hebel wiederum in eine Bewegung des Kraftübertragungselements quer zur Bewegungsrichtung des Zapfens und damit in Richtung der Längsachse des Schafts umgesetzt werden kann.

In einer weiteren bevorzugten Ausgestaltung ist das Kopplungsteil zusätzlich um eine zweite Drehachse, die quer zu der ersten Drehachse und quer zur Längsrichtung des Schafts verläuft, relativ zur Handgriffachse verschwenkbar, und sind der erste Hebel und der zweite Hebel auch um die zweite Drehachse gegeneinander verdrehbar miteinander verbunden.

Hierbei ist besonders von Vorteil, daß der Schaft bezüglich dem Handgriff mit zwei Freiheitsgraden ausgestattet ist, d.h. der Schaft kann bezüglich dem Handgriff um die erste Drehachse und zusätzlich auch um die zweite Drehachse verschiedene Winkelstellungen einnehmen. Dadurch kann das Instrument, das mit dem erfindungsgemäßen Handgriff ausgestattet ist, noch besser an die speziellen Anforderungen des das Instrument bedienenden Arztes und an die speziellen Anforderungen des jeweiligen operativen Eingriffs angepaßt werden. Somit ist die Ergonomie des erfindungsgemäßen Handgriffes noch weiter verbessert.

Dabei ist es bevorzugt, wenn zwischen dem ersten Hebel und dem zweiten Hebel ein dritter zweiarmiger Hebel angeordnet ist, wobei der erste Hebel mit dem dritten Hebel um die erste Drehachse drehbar und der zweite Hebel mit dem dritten Hebel um die zweite Drehachse drehbar verbunden ist.

Durch diese Maßnahme wird auf konstruktiv vorteilhaft einfache Weise eine Kraftübertragungsmechanik für eine Ausgestaltung des Handgriffs mit einem um zwei Drehachsen bezüglich der Handgriffachse verschwenkbaren Kopplungsteil geschaffen. Dadurch wird auch bei einem Handgriff mit zwei Freiheitsgraden des Kopplungsteiles bezüglich der Handgriffachse eine von der jeweiligen Winkelstellung des Schafts bezüglich der Handgriffachse unabhängige Kraftübertragung erzielt.

In einer weiteren bevorzugten Ausgestaltung sind der erste Hebel und/oder der zweite Hebel und/oder gegebenenfalls der dritte Hebel als etwa L-förmige Winkel ausgebildet.

Hierbei ist von Vorteil, daß die Krafteinleitung in die Hebel und die Kraftausleitung aus den Hebeln jeweils rechtwinklig zu den entsprechenden Hebelarmen der Winkel folgen kann, d.h. mit jeweils maximal möglichem Drehmoment.

In einer weiteren bevorzugten Ausgestaltung sind der erste Hebel und der zweite Hebel und gegebenenfalls der dritte Hebel hinsichtlich ihrer Hebelarmverhältnisse so ausgebildet, daß im Kraftfluß von dem beweglichen Griffteil über die Hebelanordnung auf das Kraftübertragungselement eine Kraftverstärkung eintritt.

Diese Maßnahme ist insbesondere bei solchen Instrumenten von Vorteil, mit denen hartes Gewebe im menschlichen oder tierischen Körper, beispielsweise Knochengewebe, abgetragen wird.

Durch entsprechende Wahl der Hebelarmverhältnisse können somit durch geringe Handkraft des Arztes solche harten Gewebe leicht abgetrennt werden.

Es kann jedoch auch bevorzugt sein, wenn der erste Hebel und der zweite Hebel und gegebenenfalls der dritte Hebel hinsichtlich ihrer Hebelarmverhältnisse so ausgebildet sind, daß durch die Hebelanordnung eine konstante oder geringere Kraft übertragen wird.

Dies kann beispielsweise von Vorteil sein, wenn das Instrument Werkzeuge zum Fassen von Gewebe aufweist, beispielsweise um Organe oder Gefäße im Körper zu verlagern, also bei Instrumenten, bei denen der Arzt seine Handkraft entsprechend dosieren muß, um eine Beschädigung der Gefäße oder Organe zu vermeiden.

In einer weiteren bevorzugten Ausgestaltung ist der Schaft um seine Längsachse drehbar mit dem Kopplungsteil verbindbar.

Diese Maßnahme hat den Vorteil, daß der Schaft nicht nur bezüglich dem Handgriff abwinkelbar, sondern zusätzlich auch um seine Längsachse drehbar ist, wodurch das oder die Werkzeuge am distalen Ende des Schafts bezüglich dem Handgriff zusätzlich noch in eine veränderbare Drehstellung gebracht werden können. Der erfindungsgemäße Handgriff ermöglicht bei dieser Ausgestaltung somit insgesamt drei Freiheitsgrade der Bewegung relativ zu dem Schaft, wodurch das Instrument, das mit dem Handgriff ausgestattet ist, noch besser an die jeweiligen Anforderungen des Einsatzes angepaßt werden kann.

In einer weiteren bevorzugten Ausgestaltung ist das Kraftübertragungselement direkt oder indirekt mit dem zweiten Hebel über einen Kugelzapfen-Kugelpfannen-Verbindung verbindbar.

Diese Maßnahme ist insbesondere mit der zuvor genannten Maßnahme, nach der der Schaft um seine Längsachse drehbar mit dem Kopplungsteil verbindbar ist, von Vorteil, da beim Drehen des Schafts um seine Längsachse das Kraftübertragungselement mitgedreht werden kann, da die Kugelzapfen-Kugelpfannen-Verbindung mit dem zweiten Hebel eine derartige Drehung zuläßt. Da das Kraftübertragungselement mit dem zumindest einen beweglichen Werkzeugs am distalen Ende des Schafts verbunden ist, das zumindest eine bewegliche Werkzeug andererseits im Betriebszustand des Instruments ebenfalls mit dem Schaft fest verbunden ist, ergibt sich somit eine konstruktiv einfache Bauweise, um eine Verdrehbarkeit des Schafts um seine Längsachse zu erreichen, da keine Maßnahmen getroffen werden müssen, um das Kraftübertragungselement drehbar mit dem zumindest einen beweglichen Maulteil zu verbinden.

In einer weiteren bevorzugten Ausgestaltung ist das Kopplungsteil in mehreren Winkelstellungen bezüglich zur Handgriffachse in einem Gesamtwinkelbereich von bis zu etwa 240° um die erste Drehachse und/oder um die zweite Drehachse mittels eines Rastmechanismus arretierbar.

Gemäß dieser Maßnahme kann demnach der Handgriff in einem Winkelbereich zwischen etwa -120° und etwa +120° um die erste Drehachse und/oder um die zweite Drehachse verschwenkt und in zumindest zwei, vorzugsweise jedoch einer Vielzahl von Schwenkstellungen, arretiert werden, wodurch die Ergonomie des Handgriffs hinsichtlich seiner Winkelstellung bezüglich des Schafts über einen großen Winkelbereich an die gewünschten Anforderungen des Arztes anpaßbar ist. Der Schwenkbereich kann jedoch auch kleiner sein, beispielsweise zwischen etwa -90° und +90° liegen, oder zwischen etwa 0° und +120°, um nur einige Beispiele zu nennen.

Dabei ist es bevorzugt, wenn der Rastmechanismus eine Rastnase aufweist, die mit Aussparungen des Kopplungsteils in Eingriff bringbar ist, wobei der Rastmechanismus weiterhin einen Druckknopf aufweist, so daß durch Drücken des Druckknopfes die Rastnase mit der jeweiligen Aussparung außer Eingriff bringbar ist.

Diese Art der Arretierung bzw. Entarretierung des Kopplungsteils führt vorteilhafterweise zu einer leicht bedienbaren Verschwenkbarkeit und Arretierbarkeit des Handgriffs.

In einer weiteren bevorzugten Ausgestaltung weist das Kopplungsteil eine Aufnahme für den Schaft zum lösbaren Verbinden des Schafts mit dem Kopplungsteil auf.

Hierbei ist von Vorteil, daß sich der Schaft von dem Kopplungsteil und damit von dem Handgriff abnehmen läßt, wodurch der Handgriff und der Schaft getrennt und demnach besser gereinigt werden können.

In einer weiteren bevorzugten Ausgestaltung weist das Kopplungsteil eine Aufnahme für ein proximales Ende des Kraftübertragungselements auf, wobei die Aufnahme axial beweglich ist und mit dem zweiten Hebel verbunden ist.

Bei dieser Ausgestaltung ist das Kraftübertragungselement demnach nicht direkt mit dem zweiten Hebel verbunden, sondern über eine eigens für das proximale Ende des Übertragungselements vorgesehene Aufnahme, wodurch der Vorteil erreicht wird, daß sich die Verbindung zwischen dem Kraftübertragungselement und dem zweiten Hebel besonders leicht herstellen läßt. Außerdem eröffnet diese Ausgestaltung die Möglichkeit, das Kraftübertragungselement lösbar mit dem Handgriff zu verbinden.

Dabei ist es bevorzugt, wenn die Aufnahme einen Rastmechanismus zum lösbaren Verbinden des Kraftübertragungselements mit dem Kopplungsteil aufweist.

Hierdurch wird der Vorteil erzielt, daß sich das Kraftübertragungselement von dem Kopplungsteil und damit von dem Handgriff abnehmen läßt, wodurch das Kraftübertragungselement nach dem Abnehmen von dem Handgriff leicht gereinigt werden kann.

Ein erfindungsgemäßes Instrument, das einen Schaft, zumindest ein bewegliches Werkzeug am distalen Ende des Schafts und ein axial relativ zu dem Schaft beweglich angeordnetes Kraftübertragungselement zum Betätigen des zumindest einen beweglichen Werkzeugs aufweist, ist bevorzugt und vorteilhaft mit einem erfindungsgemäßen Handgriff nach einem oder mehreren der zuvor genannten Ausgestaltungen ausgestattet.

Dabei ist es bevorzugt, wenn der Schaft und/oder das Kraftübertragungselement lösbar mit dem Handgriff verbunden ist/sind, und/oder wenn das Kraftübertragungselement lösbar mit dem Schaft verbunden ist.

Durch die lösbare Ausgestaltung sowohl des Schafts als auch des Kraftübertragungselements kann das Instrument in den Schaft, das Kraftübertragungselement und den Handgriff zerlegt werden, wodurch sich jede dieser Baugruppen besonders gut reinigen läßt.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in ihrer jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden hiernach mit Bezug auf diese näher beschrieben. Es zeigen:
- Fig. 1: eine perspektivische Gesamtansicht eines Instruments, das mit einem erfindungsgemäßen Handgriff ausgestattet ist;
- Fig. 2: den Handgriff in Fig. 1 in einer teilweise geschnittenen perspektivischen Ansicht, wobei gegenüber Fig. 1 Teile weggelassen worden sind;
- Fig. 3: eine Kraftübertragungsmechanik des Handgriffs für die Kraftübertragung von dem beweglichen Griffteil auf ein Kraftübertragungselement des Instruments in Fig. 1 in perspektivischer Ansicht in Alleinstellung;
- Fig. 4: die Kraftübertragungsmechanik in Fig. 3 in einer gegenüber Fig. 3 veränderten Perspektive;
- Fig. 5a) bis 5c): eine schematische Prinzipdarstellung der Kraftübertragungsmechanik des Handgriffs in Fig. 2, wobei Fig. 5a) eine Seitenansicht, Fig. 5b) eine Draufsicht in einer ersten Betriebsstellung der Kraftübertragungsmechanik und Fig. 5c) eine Draufsicht auf die Kraftübertragungsmechanik in einer zweiten Betriebsstellung ist;
- Fig. 6a) bis 6c): eine schematische Prinzipdarstellung einer Kraftübertragungsmechanik für einen Handgriff ist, dessen Kopplungsteil bezüglich einer Handgriffachse um zwei Drehachsen verschwenkbar ist, wobei Fig. 6a) eine perspektivische Darstellung, Fig. 6b) eine Seitenansicht und Fig. 6c) eine Draufsicht auf die Kraftübertragungsmechanik zeigt; und
- Fig. 7: einen Druckknopf des Rastmechanismus zum Verrasten des Kopplungsteils in Seitenansicht in Alleinstellung.

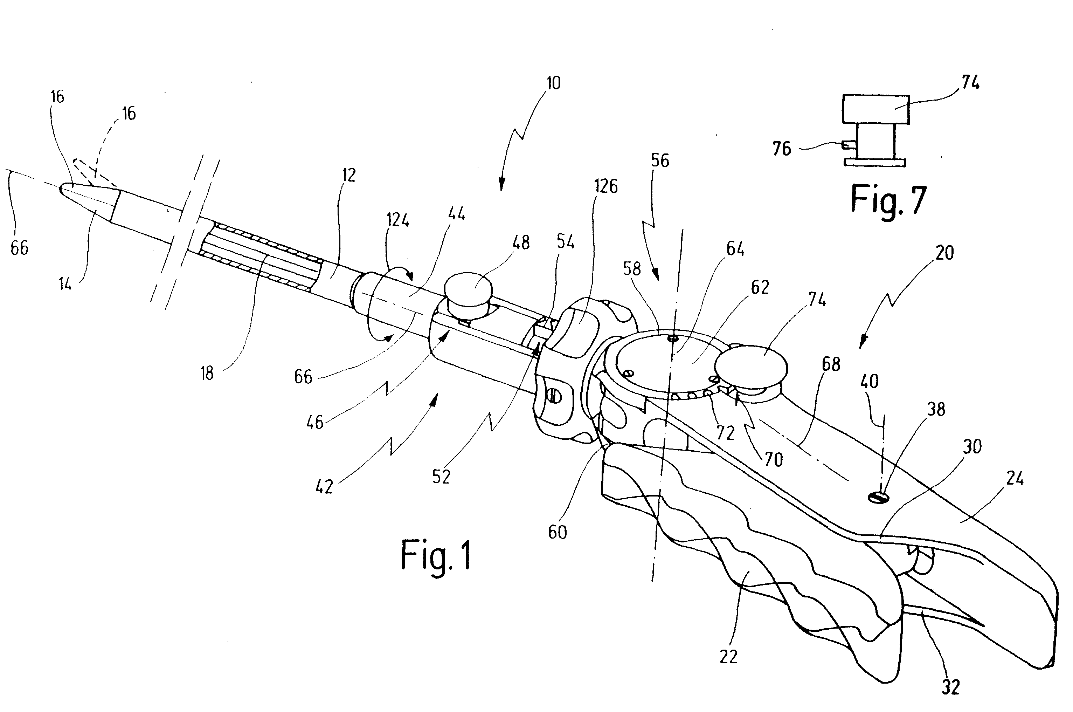

In Fig. 1 ist ein mit dem allgemeinen Bezugszeichen 10 versehenes medizinisches Instrument dargestellt, das für einen operativen Eingriff im menschlichen oder tierischen Körper beispielsweise zum Präparieren von Gewebe verwendet wird.

Das Instrument 10 weist einen lang erstreckten Schaft 12 in Form eines Rohrschafts auf, so daß das Instrument 10 als Rohrschaftinstrument bezeichnet werden kann. Am distalen Ende des Schafts 12 sind ein erstes Werkzeug 14 und ein zweites Werkzeug 16 angeordnet. Das zweite Werkzeug 16 ist relativ zu dem ersten Werkzeug 14 beweglich und zu diesem Zweck gelenkig mit dem Schaft 12 verbunden.

Das erste Werkzeug 14 und das zweite Werkzeug 16 sind als Maulteile ausgebildet, die eine Schneidfunktion zum Abtrennen von Gewebe aufweisen.

Ferner erstreckt sich in dem Schaft 12 ein Kraftübertragungselement 18, das mit dem zweiten beweglichen Werkzeug 16 kraftschlüssig verbunden ist, um dieses zum Öffnen und Schließen zu betätigen. Mit unterbrochenen Linien ist in Fig. 1 die Offenstellung des beweglichen Maulteiles 16 dargestellt. Das Kraftübertragungselement 18 ist relativ zu dem Schaft 12 axial beweglich und in dem Schaft 12 angeordnet.

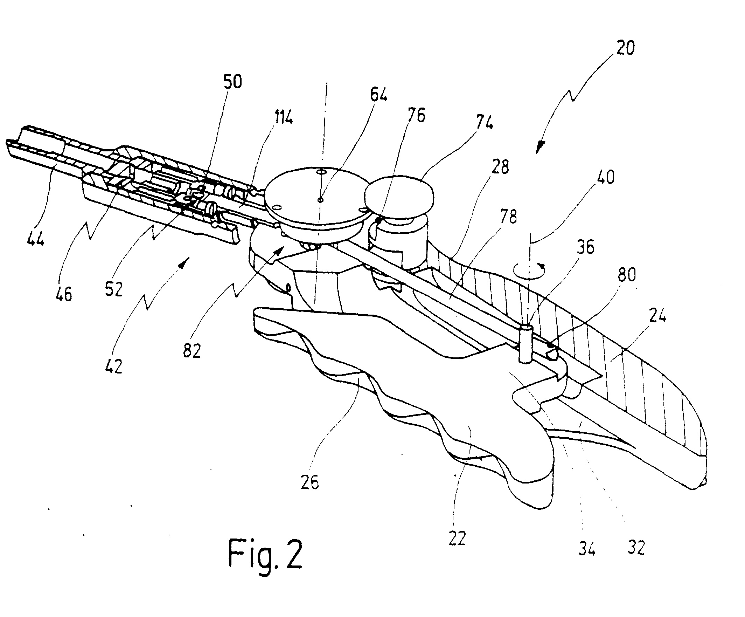

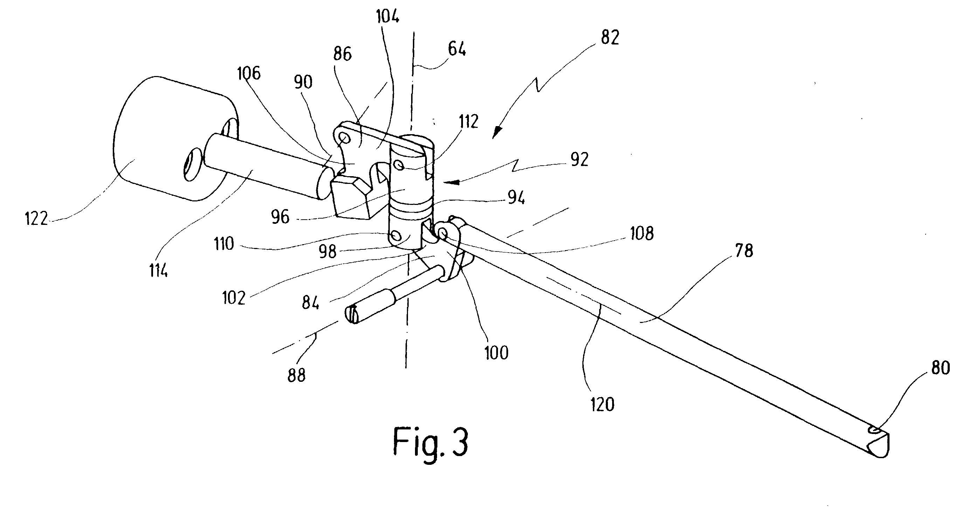

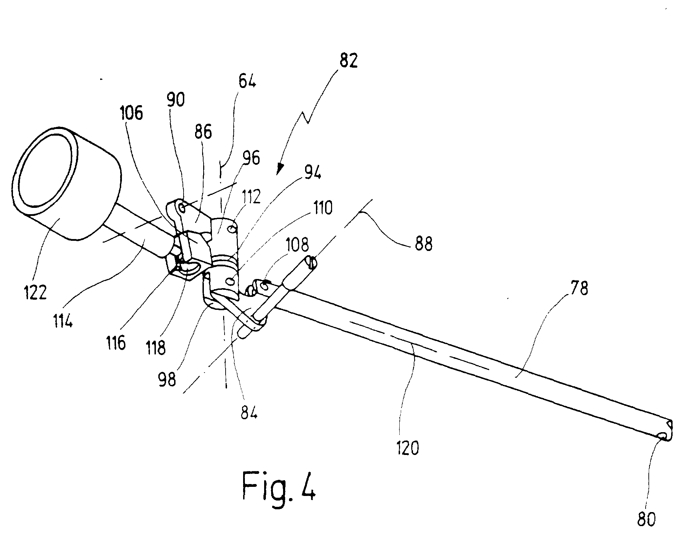

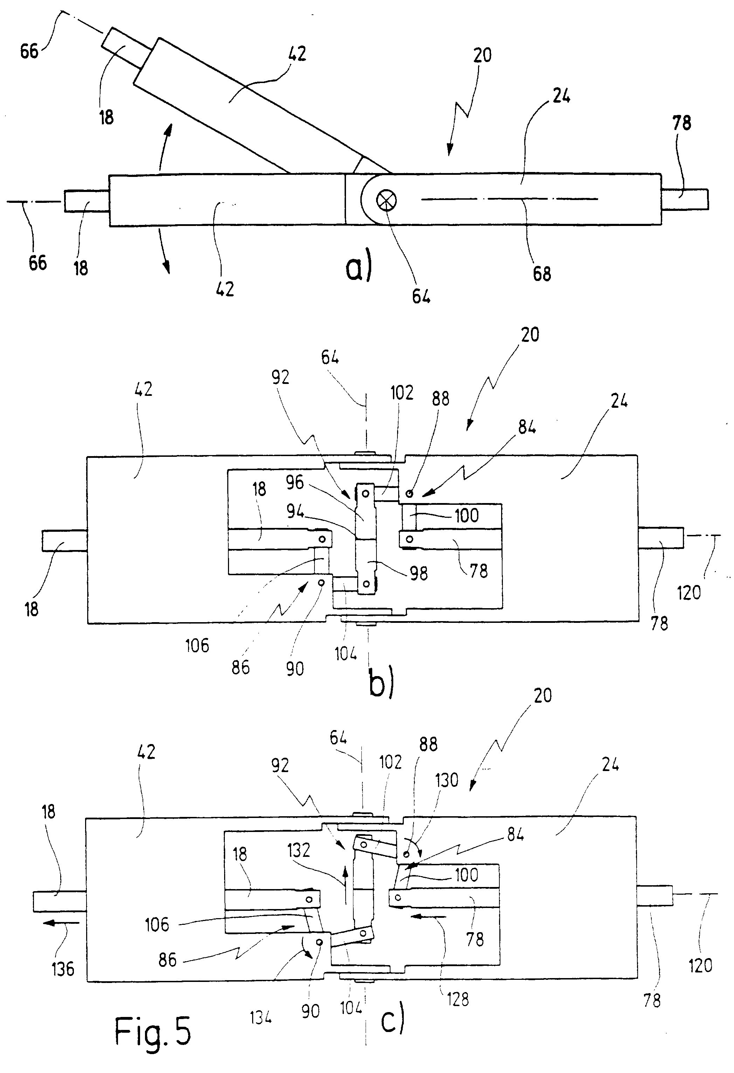

Am proximalen Ende des Schafts 12 weist das Instrument 10 einen Handgriff 20 auf, der hiernach mit Bezug auf Fig. 1 sowie mit Bezug auf Fig. 2 bis 4 näher beschrieben wird. Ferner zeigen Fig. 5a) bis 5c) schematische Prinzipskizzen der Funktionsweise der Kraftübertragungsmechanik vom Handgriff 20 auf das Kraftübertragungselement 18.

Gemäß Fig. 1 und 2 weist der Handgriff 20 ein bewegliches Griffteil 22 und ein unbewegliches Griffteil 24 auf. Das bewegliche Griffteil 22 weist vier Fingermulden 26 für Zeige-, Mittel-, Ring- und den kleinen Finger auf. Das unbewegliche Griffteil 24 weist entsprechend eine Daumenmulde 28 für den Daumen auf.

Das unbewegliche Griffteil 24 weist zwei seitliche Schenkel 30 und 32 auf, zwischen denen das bewegliche Griffteil 22, genauer gesagt ein Anlenkungsabschnitt 34 desselben angeordnet ist. Der Anlenkungsabschnitt 34 weist eine Hülse 36 auf, in die eine Schraube 38 durch die Schenkel 30 und 32 hindurch durchgesteckt und mit dem Schenkel 32 des unbeweglichen Griffteils 24 verschraubt ist, um das bewegliche Griffteil 22 und das unbewegliche Griffteil 24 unverlierbar miteinander zu verbinden.

Die Hülse 36 und die Schraube 38 definieren eine Drehachse 40, um die das bewegliche Griffteil 22 relativ zu dem unbeweglichen Griffteil 24 verschwenkbar ist.

Der Handgriff 20 weist weiterhin an seinem distalen Ende ein Kopplungsteil 42 auf, durch das der Schaft 12 mit dem Handgriff 20 verbunden bzw. verbindbar ist. Dazu ist ein proximales Endes des Schafts 12 in ein distales Rohrstück 44 des Kopplungsteils 42 eingeschoben und mittels eines Rastmechanismus 46, der einen Druckknopf 48 zum Lösen der Verrastung aufweist, mit dem Kopplungsteil 42 verrastet. Durch Drücken des Druckknopfes 48 kann die Verrastung zwischen dem Schaft 12 im Kopplungsteil 42 gelöst werden, um den Schaft 12 von dem Kopplungsteil 42 abnehmen zu können.

Das Kraftübertragungselement 18, das mit seinem distalen Ende mit dem beweglichen Werkzeug 16 kraftschlüssig verbunden ist, ist mit seinem proximalen Ende (nicht dargestellt) ebenfalls in das Rohrstück 44 des Kopplungsteils 42 eingeführt und in einer Aufnahme 50 in dem Rohrstück 44 lösbar eingehängt. Dazu weist das Kraftübertragungselement 18 am proximalen Ende eine kugelartige Verdickung auf, die formschlüssig in die Aufnahme 50 eingepaßt ist. Zum unverlierbaren Verbinden des proximalen Endes des Kraftübertragungselements 18 mit der Aufnahme 50 ist ein Rastmechanismus 52 am Kopplungsteil 42 vorgesehen, der einen Druckknopf 54 umfaßt. Durch Drücken des Druckknopfes 54 kann das Kraftübertragungselement 18 aus der Aufnahme 50 in Richtung distales Ende herausgezogen werden.

Die Aufnahme 50 ist relativ zu dem Rohrstück 44 axial verschiebbar und in seine proximale Endstellung mittels einer nicht dargestellten Feder vorgespannt.

Das Kopplungsteil 42 weist an seinem proximalen Ende einen als Gabel ausgebildeten Endabschnitt 56 auf, über den das Kopplungsteil 42 an dem unbeweglichen Griffteil 24 befestigt ist. Der Endabschnitt 56 weist einen ersten Gabelschenkel 58 sowie einen diesem gegenüberliegenden zweiten Gabelschenkel 60 auf, die beide in Form eines runden Ringes ausgebildet sind.

Der Endabschnitt 56 des Kopplungsteils 42 ist mittels einer runden Platte 62, die in den Gabelschenkel 58 eingesetzt ist, unverlierbar mit dem unbeweglichen Griffteil 24 des Handgriffs 20 verbunden. Mittels einer entsprechenden in Fig. 1 nicht sichtbaren runden Platte ist der Gabelschenkel 60 ebenfalls mit dem unbeweglichen Griffteil 24 verbunden.

Dabei ist der proximale Endabschnitt 56 des Kopplungsteils 42 um eine erste Drehachse 64, die mittig durch den proximalen Endabschnitt 56 hindurchgeht, relativ zu den Griffteilen 22 und 24 verschwenkbar, wobei die erste Drehachse 64 quer zu einer Längsachse 66 des Schafts 12 verläuft. Im vorliegenden Ausführungsbeispiels verläuft die erste Drehachse 64 senkrecht zur Längsachse 66 des Schafts 12. Durch die Verschwenkbarkeit des Kopplungsteils 42 um die erste Drehachse 64 läßt sich das Kopplungsteil 42 und damit der mit diesem verbundene Schaft 12 relativ zu einer Handgriffachse 68 in einer Vielzahl von unterschiedlichen Winkelstellungen abwinkeln. Der Schaft 12, genauer gesagt dessen Längsachse 66 kann somit zur Handgriffachse 68 wahlweise unterschiedliche Winkelstellungen einnehmen, und zwar Winkelstellungen zwischen etwa 0° und etwa 60°, so daß bei dem gezeigten Ausführungsbeispiel die maximale Winkelversatzstellung zwischen der Handgriffachse 68 und der Längsachse 66 des Schafts 12 etwa 60° beträgt. Es kann jedoch für andere Instrumente, an denen der Handgriff 20 verwendet wird, sinnvoll sein, das Kopplungsteil 42 über einen Gesamtwinkelbereich von etwa 240°, beispielsweise zwischen -120° und +120° verschwenkbar auszugestalten.

Das Kopplungsteil 42 ist in der jeweiligen gewählten Winkelstellung bezüglich der Handgriffachse 68 verrastbar, und zwar mittels eines Rastmechanismus 70. Der Rastmechanismus umfaßt eine Vielzahl von Aussparungen 72, die umfänglich verteilt am Umfangsrand des Gabelschenkels 58 des Endabschnitts 56 des Kopplungsteils 42 angeordnet sind. Der Rastmechanismus 70 umfaßt ferner einen Druckknopf 74, der am unbeweglichen Griffteil 24 angeordnet ist, und der gemäß Fig. 7 eine Rastnase 76 aufweist, die mit jeweils einer der Aussparungen 72 in Eingriff bringbar ist. Der Druckknopf 74 ist durch Federkraft in eine Stellung vorgespannt, in der die Rastnase 76 mit einer der Aussparungen 72 in Eingriff steht. Durch Herunterdrücken des Druckknopfs 74 wird die Rastnase 56 mit der Aussparung 72, in die sie gerade eingreift, außer Eingriff gebracht, so daß bei gedrücktem Druckknopf 74 das Kopplungsteil 42 um die erste Drehachse 64 relativ zur Handgriffachse 68 verschwenkt werden kann.

Im folgenden wird nun eine Kraftübertragungsmechanik des Handgriffs 20 beschrieben, mit der eine von einer Bedienungsperson auf das bewegliche Griffteil 22 ausgeübte Handkraft von dem beweglichen Griffteil 22 auf das Kraftübertragungselement 18 durch die gelenkige Verbindung des Kopplungsteils 42 mit dem unbeweglichen Griffteil 24 und dem beweglichen Griffteil 22 hindurch in jeder Winkelstellung des Kupplungsteils 42 und damit der Längsachse 66 des Schafts 12 relativ zur Handgriffachse 68 ermöglicht wird.

Die Kraftübertragungsmechanik ergibt sich aus den Fig. 2 bis 4.

An dem beweglichen Griffteil 22 ist eine axial in ihrer Längsrichtung bewegliche Koppelstange 78 mit ihrem proximalen Ende an einem Anlenkungspunkt 80 am Anlenkungsabschnitt 34 des beweglichen Griffteils 22 außerhalb der Drehachse 40 angelenkt. Eine Verschwenkung des beweglichen Griffteils um die Drehachse 40 bewirkt dann eine axiale Bewegung der Koppelstange 78 in ihrer Längsrichtung nach distal.

Über die Koppelstange 78 ist das bewegliche Griffteil 22 kraftschlüssig mit einer Hebelanordnung 82 verbunden. Die Hebelanordnung 82 weist einen ersten Hebel 84 und einen zweiten Hebel 86 auf.

Der erste Hebel 84 ist um eine handgriffeste Achse 88 verschwenkbar an dem unbeweglichen Griffteil 24 zwischen den Gabelschenkeln 58 und 60 des Endabschnitts 56 des Kopplungsteil 42 befestigt.

Der zweite Hebel 86 ist um eine kopplungsteilfeste Achse 90 an den Gabelschenkeln 58 und 60 des Endabschnitts 56 des Kopplungsteils 42 verschwenkbar befestigt, d.h., der zweite Hebel 86 wird beim Verschwenken des Kopplungsteils 42 um die erste Drehachse 64 mit verschwenkt, so daß je nach Winkelstellung des Kopplungsteils 42 zur Handgriffachse 68 die handgriffeste Achse 88 und die kopplungsteilfeste Achse 90 einen entsprechenden Winkel miteinander bilden.

Der erste Hebel 84 und der zweite Hebel 86 sind um die erste Drehachse 64 drehbar miteinander verbunden. Die drehbare Verbindung des ersten Hebels 84 mit dem zweiten Hebel 86 wird durch einen Zapfen 92 bewerkstelligt, der ein Drehgelenk 94 aufweist. Der Zapfen 92 ist aus einem ersten Zapfenteil 96 und einem zweiten Zapfenteil 98 gebildet, die über das Drehgelenk 94 um die erste Drehachse 64 gegeneinander gegensinnig verdrehbar sind.

Der Zapfen 92 selbst ist so angeordnet, daß die erste Drehachse 64 etwa mittig durch den Zapfen 92 hindurchgeht.

Der erste Hebel 84 und der zweite Hebel 86 sind jeweils als zweiarmige Hebel ausgebildet, wobei der erste Hebel 84 einen ersten Hebelarm 100 sowie einen zweiten Hebelarm 102 und der zweite Hebel 86 einen ersten Hebelarm 104 sowie einen zweiten Hebelarm 106 aufweist.

Der erste Hebel 84 und der zweite Hebel 86 sind jeweils als L-förmige Winkel ausgebildet, d.h. die Hebelarme 100 und 102 bzw. 104 und 106 stehen etwa im rechten Winkel zueinander.

Der erste Hebelarm 100 und der zweite Hebelarm 102 des ersten Hebels 84 sind etwa gleich lang, ebenso wie der erste Hebel 104 und der zweite Hebelarm 106 des zweiten Hebels 86.

Die Koppelstange 78 ist an dem ersten Hebelarm 100 des ersten Hebels 84 an einem Anlenkungspunkt 108 angelenkt.

Der zweite Hebelarm 102 des ersten Hebels 84 ist an dem Zapfenteil 98 des Zapfens 92 an einem Anlenkungspunkt 110 angelenkt. Der erste Hebelarm 104 des zweiten Hebels 86 ist an dem Zapfenteil 96 des Zapfens 92 an einem Anlenkungspunkt 112 angelenkt.

Mit dem zweiten Hebelarm 106 des zweiten Hebels 86 ist eine weitere Koppelstange 114 verbunden, und zwar über eine Kugelzapfen-Kugelpfannen-Verbindung, wobei ein proximales Ende der weiteren Koppelstange 114 einen Kugelzapfen 116 aufweist, der unverlierbar in einer Kugelpfanne 118 des zweiten Hebelarms 106 des zweiten Hebels 86 aufgenommen ist. Die Koppelstange 114 ist gemäß Fig. 2 kraftschlüssig mit der Aufnahme 50 verbunden, in die das proximale Ende des Kraftübertragungselements 18 eingehängt ist. Die Koppelstange 114 steht somit stets in gradliniger Verlängerung mit dem Kraftübertragungselement 18, bildet jedoch mit einer Längsachse 120 der Koppelstange 78 je nach der eingestellten Winkelstellung zwischen dem Kopplungsteil 42 und der Handgriffachse 68 einen unterschiedlichen Winkel. Die Hebelanordnung 82 ermöglicht nun eine Kraftübertragung durch den Winkelscheitel hindurch.

Die weitere Koppelstange 114 ist durch eine ortsfeste Hülse 122 in das Rohrstück 44 des Kopplungsteils 42 geführt und dort mit der Aufnahme 50 für das Kraftübertragungselement 18 verbunden.

Die Koppelstange 78 ist im Handgriff 20 so angeordnet, daß ihre Längsachse 120 stets auf einer Geraden liegt, die die erste Drehachse 64 schneidet, so daß die Koppelstange 78 kein Drehmoment auf die Hebelanordnung 82 bezüglich der ersten Drehachse 64 ausübt. Ebenso ist die weitere Koppelstange 114 so angeordnet, daß sie auf die Drehachse 64 hin gerichtet ist.

Wieder mit Bezug auf Fig. 1 ist das Rohrstück 44 des Kopplungsteils 42 ferner um die Längsachse 66, d.h. somit um die Längsachse des Rohrstücks 44 relativ zu dem Endabschnitt 56 des Kopplungsteils 42 gemäß einem Doppelpfeil 124 verdrehbar. Die Verdrehbarkeit des Rohrstücks 44 und damit des Schafts 12 beträgt 360° in beiden Drehrichtungen um die Längsachse 66 des Schafts 12. Zum Verdrehen des Rohrstücks 44 ist an dem Rohrstück 44 ferner ein Stellrad 126 angeordnet, das mit dem Daumen zum Verdrehen des Schafts 12 bedienbar ist. Beim Verdrehen des Rohrstücks 44 und damit des Schafts 12 um die Längsachse 66 des Schafts 12 wird das Kraftübertragungselement 18 mitgedreht, was durch die Kugelzapfen-Kugelpfannen-Verbindung der weiteren Koppelstange 114 mit dem zweiten Hebelarm 106 des zweiten Hebels 86 ermöglicht wird.

Mit Bezug auf Fig. 5a) bis 5c) wird nun die Funktionsweise der Kraftübertragungsmechanik des Handgriffs 20 näher beschrieben. Fig. 5a) bis 5c) zeigen den Handgriff 20 in einer äußerst schematischen Darstellung, wobei in der schematischen Darstellung das unbewegliche Griffteil 24 und das Kopplungsteil 42 zur Vereinfachung als Blockbilder dargestellt sind.

Fig. 5a) zeigt zunächst die Verschwenkbarkeit des Kopplungsteils 42 relativ zur Handgriffachse 68 um die erste Drehachse 64. Beim Verschwenken des Kopplungsteils 42 um die erste Drehachse 64 wird das erste Zapfenteil 96 relativ zum zweiten Zapfenteil 98 des Zapfens 92 ebenfalls um die erste Drehachse 64 verdreht, ohne daß das Zapfenteil 96 von dem Zapfenteil 98 getrennt wird. Dies bedeutet, daß auch während des Verschwenkens des Kopplungsteils 42 um die erste Drehachse 64 eine kraftschlüssige Verbindung der Koppelstange 78 mit dem Kraftübertragungselement 18 besteht. Daher kann auch während eines operativen Eingriffs, bei dem das bewegliche Werkzeug 16 durch Betätigen des beweglichen Griffteils 22 bewegt wird, gleichzeitig der Schaft 12 bezüglich der Handgriffachse 68 in eine andere Winkelstellung gebracht werden.

Fig. 5b) zeigt den Ruhezustand der Kraftübertragungsmechanik, d.h. bei geschlossenen Werkzeugen 14 und 16 in Fig. 1. Wird ausgehend von dieser Ruhestellung das bewegliche Griffteil 22 betätigt, wird die Koppelstange 78 in Richtung ihrer Längsachse 120 in Richtung eines Pfeils 128 nach distal verschoben. Die Koppelstange 78, die am ersten Hebelarm 100 des ersten Hebels 84 angreift, verschwenkt den ersten Hebel 84 um die handgriffeste Achse 88 in Richtung eines Pfeiles 130. Beim Verschwenken des ersten Hebels 84 bewegt der zweite Hebelarm 102 des ersten Hebels 84 den Zapfen 92 in Richtung eines Pfeiles 132, d.h. in Richtung seiner Längsachse bzw. in Richtung der ersten Drehachse 64. Da der Zapfen 92 auch am ersten Hebelarm 104 des zweiten Hebels 86 angreift, wird der zweite Hebel 86 um die kopplungsteilfeste Achse 90 in Richtung eines Pfeiles 134 verschwenkt.

Der zweite Hebelarm 106 des zweiten Hebels 86, der mit dem Kraftübertragungselement 18 kraftschlüssig verbunden ist, bewegt das Kraftübertragungselement 18 demnach in Richtung eines Pfeiles 136 ebenfalls nach distal, wodurch das bewegliche Werkzeug 16 am distalen Ende des Schafts 12 bewegt wird. Beim Loslassen des beweglichen Griffteils 22 bewirkt die Vorspannung der Aufnahme 50 für das Kraftübertragungselement 18, das die Kraftübertragungsmechanik wieder in ihre in Fig. 5b) dargestellte Ruhelage selbsttägig zurückkehrt.

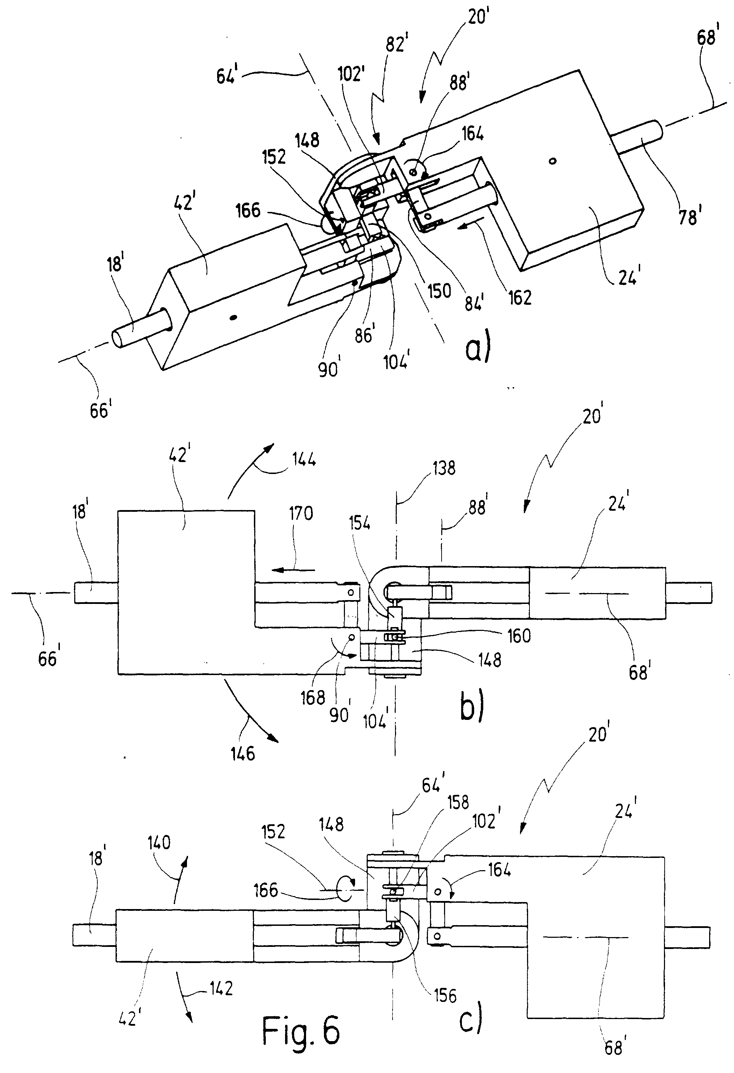

In Fig. 6a) bis 6c) ist ein weiteres Ausführungsbeispiel eines Handgriffs 20' in einer den Fig. 5a) bis 5c) vergleichbaren äußerst schematischen Darstellung gezeigt. Vergleichbare Teile wurden mit den gleichen Bezugszeichen mit hochgestelltem ' versehen.

Bei dem Handgriff 20' ist wiederum ein Kopplungsteil 42' um eine erste Drehachse 64' relativ zu einer Handgriffachse 68' verschwenkbar.

Das Kopplungsteil 42' ist zusätzlich noch um eine zweite Drehachse 138 relativ zur Handgriffachse 68' verschwenkbar, wobei die zweite Drehachse 138 quer zur ersten Drehachse 64' und quer zu einer Längsachse 66' des Schafts (Schaft nicht dargestellt) verläuft. Das Kopplungsteil 42' läßt sich somit in Richtung von Pfeilen 140 und 142 um die zweite Drehachse 138 und in Richtung von Pfeilen 144 und 146 um die erste Drehachse 64' relativ zur Handgriffachse 68' verschwenken.

Gegenüber dem Kopplungsteil 42 ist das Kopplungsteil 42' durch ein Zwischenstück 148 modifiziert, das das Kopplungsteil 42' einerseits um die erste Drehachse 64' und andererseits um die zweite Drehachse 138 gelenkig mit dem unbeweglichen Griffteil 24' verbindet.

Die Kraftübertragungsmechanik von einer Koppelstange 78', die der Koppelstange 78 im vorherigen Ausführungsbeispiel entspricht, auf ein Kraftübertragungselement 18', das dem Kraftübertragungselement 18 im vorherigen Ausführungsbeispiel entspricht, ist ebenfalls geringfügig modifiziert, wie hiernach beschrieben wird.

Das Kraftübertragungselement 18' und die Koppelstange 78' sind wiederum über eine Hebelanordnung 82' miteinander verbunden, die einen ersten Hebel 84', der mit der Koppelstange 78' verbunden ist, und einen zweiten Hebel 86' aufweist, der mit dem Kraftübertragungselement 18' verbunden ist. Der erste Hebel 84' und der zweite Hebel 86' sind wiederum als zweiarmige Hebel in Form von L-förmigen Winkeln ausgebildet.

Der erste Hebel 84' und der zweite Hebel 86' sind wiederum um die erste Drehachse 64' drehbar miteinander verbunden.

Der erste Hebel 84' ist um eine handgriffeste Achse 88' und der zweite Hebel 86' um eine kopplungsteilfeste Achse 90' verschwenkbar gelagert.

Der erste Hebel 84' und der zweite Hebel 86' sind außer um die erste Drehachse 64' auch um die zweite Drehachse 138 drehbar miteinander verbunden.

Die Verbindung zwischen dem ersten Hebel 84' und dem zweiten Hebel 86' wird durch einen dritten, ebenfalls zweiarmig ausgebildeten Hebel 150 bewerkstelligt, der um eine zwischenstückfeste Achse 152 verschwenkbar an dem Zwischenstück 148 gelagert ist.

Der dritte Hebel 150 weist zwei etwa rechtwinklig zueinander stehende Hebelarme 154 und 156 auf, wobei der Hebelarm 154 an seinem freien Ende einen Kugelzapfen 158 und der Hebelarm 156 an seinem freien Ende einen Kugelzapfen 160 aufweist.

Der Kugelzapfen 158 des Hebelarms 154 greift in einen endseitig geschlitzten zweiten Hebelarm 102' des ersten Hebels 84' ein und ist in diesem um die erste Drehachse 64' relativ zu dem ersten Hebel 84' drehbar.

Der Kugelzapfen 160 des zweiten Hebelarms 156 greift in einen endseitig geschlitzt ausgeführten ersten Hebelarm 104' des zweiten Hebels 86' ein, und ist in diesem um die zweite Drehachse 138 relativ zu dem zweiten Hebel 86' drehbar. Durch diese Verbindung des dritten Hebels 150 mit dem ersten Hebel 84' und dem zweiten Hebel 86' wird gewährleistet, daß das Kopplungsteil 42' ohne Trennung der kraftschlüssigen Verbindung der Koppelstange 78' mit dem Kraftübertragungselement 18' um die erste Drehachse 64' bzw. um die zweite Drehachse 138 verschwenkt werden kann.

Auch bei diesem Ausführungsbeispiel ist die Koppelstange 78' so angeordnet, daß sie auf einer Geraden liegt, die sowohl die erste Drehachse 64' als auch die zweite Drehachse 138 schneidet, so daß die Koppelstange 78' weder ein Drehmoment auf das Kopplungsteil 42' in Bezug auf die erste Drehachse 64' noch in Bezug auf die zweite Drehachse 138 ausübt.

Wird nun die Koppelstange 78' in Richtung eines Pfeiles 162 axial bewegt, wird der erste Hebel 84' um die handgriffeste Achse 88' in Richtung eines Pfeiles 164 verschwenkt. Der zweite Hebelarm 102' des ersten Hebels 84', der an dem ersten Hebelarm 154 des dritten Hebels 150 angreift, verschwenkt den dritten Hebel um die zwischenstückfeste Achse 152 in Richtung eines Pfeiles 166 (in Fig. 6a) und 6c) dargestellt).

Der zweite Hebelarm 156 des dritten Hebels 150, der an dem zweiten Hebel 86' angreift, verschwenkt diesen um die kopplungsteilfeste Achse 90' in Richtung eines Pfeils 168, wodurch das Kraftübertragungselement 18' in Richtung eines Pfeiles 170 axial nach distal verschoben wird.

Bei den Hebelanordnungen 82 in dem Ausführungsbeispiel gemäß Fig. 1 bis 5 sowie bei der Hebelanordnung 82' gemäß dem Ausführungsbeispiel in Fig. 6 sind die in die jeweilige Hebelanordnung eingehenden Hebel mit solchen Hebelarmverhältnissen ausgebildet, daß durch die Hebelanordnung 82' bzw. 82 eine konstante Kraft von der Koppelstange 78' bzw. 78 auf das Kraftübertragungselement 18 bzw. 18' übertragen wird.

Es ist jedoch auch denkbar, die einzelnen Hebel der Hebelanordnung 82 und 82' mit solchen Hebelarmverhältnissen auszubilden, daß im Kraftfluß von der Koppelstange 78 bzw. 78' über die Hebelanordnung 82 bzw. 82' auf das Kraftübertragungselement 18 bzw. 18' eine Kraftverstärkung eintritt.

Wieder mit Bezug auf Fig. 1 ist das Instrument 10 schließlich in den Handgriff 20, den Schaft 12, und das Kraftübertragungselement 18 zerlegbar. Die lösbare Verbindung zwischen dem Schaft 12 und dem Handgriff 20 sowie zwischen dem Kraftübertragungselement 18 und dem Handgriff 20 wurde vorstehend bereits beschrieben. Ferner ist jedoch auch das Kraftübertragungselement 18 von dem Schaft 12 abnehmbar, indem diese beiden Teile mittels eines bajonettartigen Verschlusses im Bereich der Werkzeuge 14, 16 miteinander lösbar verbunden sind.

Während das Kraftübertragungselement 18 bei den zuvor beschriebenen Ausführungsbeispielen auf Schub arbeitet, kann der erfindungsgemäße Kraftübertragungsmechanismus mit geringfügigen Modifikationen leicht auf den Fall angepaßt werden, daß das Kraftübertragungselement 18 auf Zug arbeitet. Ebenso kann die Kraftübertragung von dem beweglichen Griffteil 22 auf die Hebelanordnung 82 anstatt, wie zuvor beschrieben, durch eine Schubbewegung der Koppelstange 78 auch durch eine Zugbewegung der Koppelstange 78 durch eine entsprechende Modifikation ersetzt werden.

Im übrigen läßt sich die Erfindung auch bei scherenartigen Griffteilen vorteilhaft einsetzen.

## Patentansprüche

1. Handgriff für ein medizinisches Instrument (10), mit zumindest einem beweglichen Griffteil (22), und mit einem Kopplungsteil (42; 42'), durch das ein Schaft (12) mit dem Handgriff verbindbar ist, wobei das bewegliche Griffteil (22) mit einem in Richtung einer Längsachse (66; 66') des Schafts (12) axial beweglichen Kraftübertragungselement (18; 18') verbindbar ist, um eine Bewegung des zumindest einen beweglichen Griffteils (22) in eine axiale Bewegung des Kraftübertragungselements (18; 18') zu übertragen, wobei das Kopplungsteil (42; 42') um zumindest eine erste quer zur Längsachse (66; 66') des Schafts (12) verlaufende Drehachse (64; 64') relativ zu einer Handgriffachse (68; 68') verschwenkbar ist, **dadurch gekennzeichnet, daß** das bewegliche Griffteil (22) mit dem Kraftübertragungselement (18; 18') über eine Hebelanordnung (82; 82') verbindbar ist, die zumindest zwei zweiarmige Hebel (84, 86; 84', 86') aufweist, von denen ein erster Hebel (84; 84') um eine handgriffeste Achse (88; 88') und ein zweiter Hebel (86; 86') um eine kopplungsteilfeste Achse (90; 90') schwenkbar ist, und daß die beiden Hebel (84, 86; 84', 86') kraftschlüssig und um die erste Drehachse (64; 64') gegeneinander verdrehbar miteinander verbunden sind.

2. Handgriff nach Anspruch 1, **dadurch gekennzeichnet, daß** das bewegliche Griffteil (22) mit einer in ihrer Längsrichtung axial verschiebbaren Koppelstange (78; 78') verbunden ist, die mit einem ersten Hebelarm (100) des ersten Hebels (84; 84') verbunden ist.

3. Handgriff nach Anspruch 2, **dadurch gekennzeichnet, daß** die Koppelstange (78; 78') so angeordnet ist, daß ihre Längsachse (120) auf einer Geraden liegt, die die erste Drehachse (64; 64') schneidet.

4. Handgriff nach einem der Ansprüche 1 bis 3, **dadurch gekennzeichnet, daß** der erste Hebel (84) und der zweite Hebel (86) durch einen Zapfen (92) miteinander verbunden sind, der ein Drehgelenk (94) aufweist, dessen Drehachse mit der ersten Drehachse (64) zusammenfällt.

5. Handgriff nach Anspruch 4, **dadurch gekennzeichnet, daß** der erste Hebel (84) eine axiale Bewegung der Koppelstange (78) in eine axiale Bewegung des Zapfens (92) in Richtung der ersten Drehachse (64) umsetzt, und daß der zweite Hebel (86) die axiale Bewegung des Zapfens (92) in eine axiale Bewegung des Kraftübertragungselements (18) in Richtung der Längsachse (66) des Schafts (12) umsetzt.

6. Handgriff nach einem der Ansprüche 1 bis 3, **dadurch gekennzeichnet, daß** das Kopplungsteil (42) zusätzlich um eine zweite Drehachse (138), die quer zu der ersten Drehachse (64') und quer zur Längsrichtung (66') des Schafts (12) verläuft, relativ zur Handgriffachse (68') verschwenkbar ist, und daß der erste Hebel (84') und der zweite Hebel (86') auch um die zweite Drehachse (138) gegeneinander verdrehbar miteinander verbunden sind.

7. Handgriff nach Anspruch 6, **dadurch gekennzeichnet, daß** zwischen dem ersten Hebel (84') und dem zweiten Hebel (86') ein dritter zweiarmiger Hebel (150) angeordnet ist, wobei der erste Hebel (84') mit dem dritten Hebel (150) um die erste Drehachse (64') drehbar und der zweite Hebel (86') mit dem dritten Hebel (150') um die zweite Drehachse (138) drehbar verbunden ist.

8. Handgriff nach einem der Ansprüche 1 bis 7, **dadurch gekennzeichnet, daß** der erste Hebel (84; 84') und/oder der zweite Hebel (86; 86') und/oder gegebenenfalls der dritte Hebel (150) als etwa L-förmige Winkel ausgebildet sind.

9. Handgriff nach einem der Ansprüche 1 bis 8, **dadurch gekennzeichnet, daß** der erste Hebel (84; 84') und der zweite Hebel (86; 86') und gegebenenfalls der dritte Hebel (150) hinsichtlich ihrer Hebelarmverhältnisse so ausgebildet sind, daß im Kraftfluß von dem beweglichen Griffteil (22) über die Hebelanordnung (82; 82') auf das Kraftübertragungselement (18; 18') eine Kraftverstärkung eintritt.

10. Handgriff nach einem der Ansprüche 1 bis 8, **dadurch gekennzeichnet, daß** der erste Hebel (84; 84') und der zweite Hebel (86; 86') und gegebenenfalls der dritte Hebel (150) hinsichtlich ihrer Hebelarmverhältnisse so ausgebildet sind, daß durch die Hebelanordnung (82; 82') eine konstante oder geringere Kraft übertragen wird.

11. Handgriff nach einem der Ansprüche 1 bis 10, **dadurch gekennzeichnet, daß** der Schaft (12) um seine Längsachse (66) drehbar mit dem Kopplungsteil (42) verbindbar ist.

12. Handgriff nach einem der Ansprüche 1 bis 11, **dadurch gekennzeichnet, daß** das Kraftübertragungselement (18) direkt oder indirekt mit dem zweiten Hebel (86) über eine Kugelzapfen - Kugelpfannen - Verbindung verbindbar ist.

13. Handgriff nach einem der Ansprüche 1 bis 12, **dadurch gekennzeichnet, daß** das Kopplungsteil (42) in mehreren Winkelstellungen bezüglich zur Handgriffachse (68) in einem Gesamtwinkelbereich von bis zu etwa 240° um die erste Drehachse (64) und/oder um die zweite Drehachse (138) mittels eines Rastmechanismus (70) arretierbar ist.

14. Handgriff nach Anspruch 13, **dadurch gekennzeichnet, daß** der Rastmechanismus (70) eine Rastnase (76) aufweist, die mit Aussparungen (72) des Kopplungsteils (42) in Eingriff bringbar ist, wobei der Rastmechanismus (70) weiterhin einen Druckknopf (74) aufweist, so daß durch Drücken des Druckknopfes (74) die Rastnase (76) mit der jeweiligen Aussparung (72) außer Eingriff bringbar ist.

15. Handgriff nach einem der Ansprüche 1 bis 14, **dadurch gekennzeichnet, daß** das Kopplungsteil (42) eine Aufnahme für den Schaft (12) zum lösbaren Verbinden des Schafts (12) mit dem Kopplungsteil (42) aufweist.

16. Handgriff nach einem der Ansprüche 1 bis 15, **dadurch gekennzeichnet, daß** das Kopplungsteil (42) eine Aufnahme (50) für ein proximales Ende des Kraftübertragungselements (18) aufweist, wobei die Aufnahme (50) axial beweglich ist und mit dem zweiten Hebel (86) verbunden ist.

17. Handgriff nach Anspruch 16, **dadurch gekennzeichnet, daß** die Aufnahme (50) einen Rastmechanismus (52) zum lösbaren Verbinden des Kraftübertragungselements (18) mit dem Kopplungsteil (42) aufweist.

18. Medizinisches Instrument, mit einem Schaft (12), mit zumindest einem beweglichen Werkzeug (16) am distalen Ende des Schafts (12), mit einem axial in dem Schaft (12) beweglich angeordneten Kraftübertragungselement (18; 18') zum Betätigen des zumindest einen beweglichen Werkzeugs (16), **gekennzeichnet durch** einen Handgriff (20; 20') nach einem der Ansprüche 1 bis 17.

19. Instrument nach Anspruch 18, **dadurch gekennzeichnet, daß** der Schaft (12) lösbar mit dem Handgriff (20; 20') verbunden ist.

20. Instrument nach Anspruch 18 oder 19, **dadurch gekennzeichnet, daß** das Kraftübertragungselement (18; 18') lösbar mit dem Handgriff (20; 20') verbunden ist.

21. Instrument nach einem der Ansprüche 18 bis 20, **dadurch gekennzeichnet, daß** das Kraftübertragungselement (18; 18') lösbar mit dem Schaft (12) verbunden ist.

## Claims

1. Handle for a medical instrument (10), comprising at least one moveable grip element (22) and a coupling portion (42; 42') through which a shaft (12) can be connected to the handle, wherein the moveable grip element (22) can be connected to a force transmission element (18; 18') axially moveable in direction of a longitudinal axis (66; 66') of the shaft (12) to transfer motion of the at least one moveable grip element (22) into axial motion of the force transmission element (18; 18'), wherein the coupling portion (42; 42') is pivotal relative to a handle axis (68; 68') about at least one first pivot axis (64; 64') running transversely to the longitudinal axis (66; 66') of the shaft (12), **characterized in that** the moveable grip element (22) can be connected to the force transmission element (18; 18') through a lever arrangement (82; 82'), which comprises at least two double-arm levers (84, 86; 84', 86'), the first lever (84; 84') of which is pivotal about an axis (88; 88') stationary with respect to the handle and the second lever (86; 86') of which is pivotal about an axis (90; 90') stationary with respect to the coupling portion, and the two levers (84, 86; 84', 86') are connected to one another in force-locked manner and rotatable with respect to one another about the first pivot axis (64; 64').

2. The handle of claim 1, **characterized in that** the moveable grip element (22) is connected to a coupling rod (78; 78') axially displaceable along its longitudinal direction, which is connected to a first lever arm (100) of the first lever (84; 84').

3. The handle of claim 2, **characterized in that** the coupling rod (78; 78') is arranged such that its longitudinal axis (120) lies along a line intersecting the first pivot axis (64; 64').

4. The handle of anyone of claims 1 through 3, **characterized in that** the first lever (84) and the second lever (86) are connected to one another by a journal (92) having a pivot joint (94) whose pivot axis is coincident with the first pivot axis (64).

5. The handle of claim 4, **characterized in that** the first lever (84) translates axial motion of the coupling rod (78) into axial motion of the journal (92) in the direction of the first pivot axis (64) and wherein the second lever (86) translates the axial motion of the journal (92) into axial motion of the force transmission element (18) in the direction of the longitudinal axis (66) of the shaft (12).

6. The handle of anyone of claims 1 through 3, **characterized in that** the coupling portion (42) additionally is pivotal relative to the handle axis (68') about a second pivot axis (138), which runs transversely to the first pivot axis (64') and transversely to the longitudinal axis (66') of the shaft (12), and the first lever (84') and the second lever (86') are also connected to one another to be rotatable with respect to one another about the second pivot axis (138).

7. The handle of claim 6, **characterized in that** a third double-arm lever (150) is arranged between the first lever (84') and the second lever (86'), wherein the first lever (84') is connected with the third lever (150) rotatably about the first pivot axis (64') and the second lever (86') is connected with the third lever (150) rotatably about the second pivot axis (138).

8. The handle of anyone of claims 1 through 7, **characterized in that** the first lever (84; 84') and/or the second lever (86; 86') and/or optionally the third lever (150) are formed approximately as an L-shaped angle.

9. The handle of anyone of claims 1 through 8, **characterized in that** the first lever (84; 84') and the second lever (86; 86') and optionally the third lever (150) are configured with respect to their lever arm ratios such that a force enhancement results in the force transfer from the moveable grip element (22) through the lever arrangement (82; 82') to the force transmission element (18; 18').

10. The handle of anyone of claims 1 through 8, **characterized in that** the first lever (84; 84') and the second lever (86; 86') and optionally the third lever (150) are configured with respect to their lever arm ratios such that a constant or smaller force is transferred through the lever arrangement (82; 82').

11. The handle of anyone of claims 1 through 10, **characterized in that** the shaft (12) can be connected with the coupling portion (42) to rotate about its longitudinal axis (66).

12. The handle of anyone of claims 1 through 11, **characterized in that** the force transmission element (18) can be directly or indirectly connected to the second lever (86) in a ball and socket connection.

13. The handle of anyone of claims 1 through 12, **characterized in that** the coupling portion (42) can be locked by means of a snap-locking mechanism (70) in a plurality of angular positions with respect to the handle axis (68) in a total angular range of up to about 240° about the first pivot axis (64) and/or about the second pivot axis (138).

14. The handle of claim 13, **characterized in that** the snap-locking mechanism (79) comprises a locking nose (76) engageable with notches (72) of the coupling portion (42), wherein the snap-locking mechanism (72) further comprises a button (74), such that the locking nose (76) is disengageable from the respective notch (72) by depressing the button (74).

15. The handle of anyone of claims 1 through 14, **characterized in that** the coupling portion (42) comprises a seat for the shaft (12) for releaseably connecting the shaft (12) with the coupling portion (42).

16. The handle of anyone of claims 1 through 15, **characterized in that** the coupling portion (42) comprises a seat (50) for the proximal end of the force transmission element (18), wherein the seat (50) is axially moveable and connected to the second lever (86).

17. The handle of claim 16, **characterized in that** the seat (50) comprises a snap-locking mechanism (52) for releaseably connecting the force transmission element (18) with the coupling portion (42).

18. Medical instrument, comprising a shaft (12) with at least one moveable tool (16) at the distal end of the shaft (12), and a force transmission element (18; 18') arranged to be axially moveable in the shaft (12) for actuating the at least one moveable tool (16), **characterized by** a handle (20; 20') of anyone of claims 1 through 17.

19. The instrument of to claim 18, **characterized in that** the shaft (12) is releaseably connected to the handle (20; 20').

20. The instrument of claim 18 or 19, **characterized in that** the force transmission element (18; 18') is releaseably connected to the handle (20; 20').

21. The instrument of anyone of claims 18 through 20, **characterized in that** the force transmission element (18; 18') is releaseably connected to the shaft (12).

## Revendications

1. Poignée pour un instrument médical (10), comportant au moins une partie de poignée déplaçable (22) ainsi qu'une partie d'accouplement (42 ; 42') au moyen de laquelle une tige (12) peut être reliée à la poignée, la partie de poignée déplaçable (22) pouvant être reliée à un élément (18; 18') de transmission des forces, déplaçable axialement en direction d'un axe longitudinal (66 ; 66') de la tige (12), pour transmettre un mouvement de la ou des parties de poignée déplaçables (22) en un mouvement axial de l'élément (18; 18') de transmission des forces, la partie d'accouplement (42 ; 42') pouvant pivoter par rapport à un axe de poignée (68; 68'), autour d'au moins un premier axe de rotation (64 ; 64') s'étendant perpendiculairement à l'axe longitudinal (66; 66') de la tige (12), **caractérisée en ce que** la partie de poignée déplaçable (22) peut être reliée à l'élément (18 ; 18') de transmission des forces par l'intermédiaire d'un dispositif à leviers (82; 82') qui comporte au moins deux leviers (84; 86; 84', 86') à deux bras, dont un premier levier (84; 84') peut pivoter autour d'un axe (88; 88') solidaire de la poignée, et un deuxième levier (86; 86') peut pivoter autour d'un axe (90; 90') solidaire de la partie d'accouplement, et **en ce que** les deux leviers (84, 86; 84', 86') sont reliés entre eux à force et peuvent tourner l'un par rapport à l'autre autour du premier axe de rotation (64 ; 64').

2. Poignée selon la revendication 1, **caractérisée en ce que** la partie de poignée déplaçable (22) est reliée à une tige d'accouplement (78; 78') qui peut coulisser axialement dans sa direction longitudinale et qui est reliée à un premier bras de levier (100) du premier levier (84; 84').

3. Poignée selon la revendication 2, **caractérisée en ce que** la tige d'accouplement (78; 78') est disposée de manière que son axe longitudinal (120) se situe sur une droite qui coupe le premier axe de rotation (64 ; 64').

4. Poignée selon l'une des revendications 1 à 3, **caractérisée en ce que** le premier levier (84) et le deuxième levier (86) sont reliés entre eux par un tenon (92) qui comporte une articulation tournante (94) dont l'axe de rotation coïncide avec le premier axe de rotation (64).

5. Poignée selon la revendication 4, **caractérisée en ce que** le premier levier (84) convertit un mouvement axial de la tige d'accouplement (78) en un mouvement axial du tenon (92), en direction du premier axe de rotation (64, et **en ce que** le deuxième levier 86 convertit le mouvement axial du tenon 92 en un mouvement axial de l'élément (18) de transmission des forces, en direction de l'axe longitudinal (66) de la tige (12).

6. Poignée selon l'une des revendications 1 à 3, **caractérisée en ce que** la partie d'accouplement (42) peut pivoter en outre par rapport à l'axe de poignée (68'), autour d'un deuxième axe de rotation (138) qui s'étend perpendiculairement au premier axe de rotation (64') et perpendiculairement à la direction longitudinale (66') de la tige (12), et **en ce que** le premier levier (84') et le deuxième levier (86') sont reliés entre eux aussi de manière à pouvoir tourner l'un par rapport à l'autre, autour du deuxième axe de rotation (138).

7. Poignée selon la revendication 6, **caractérisée en ce qu'**un troisième levier (150) à deux bras est disposé entre le premier levier (84') et le deuxième levier (85'), le premier levier (84') étant relié au troisième levier (150) de manière à pouvoir tourner autour du premier axe de rotation (64') et le deuxième levier (86') étant relié au troisième levier (150'), de manière à pouvoir tourner autour du deuxième axe de rotation (138).

8. Poignée selon l'une des revendications 1 à 7, **caractérisée en ce que** le premier levier (84 ; 84') et/ou le deuxième levier (86 ; 86') et/ou éventuellement le troisième levier (150) sont réalisés en tant que cornières approximativement en L.

9. Poignée selon l'une des revendications 1 à 8, **caractérisée en ce que** le premier levier (84 ; 84') et le deuxième levier (86 ; 86') et éventuellement le troisième levier (150) sont réalisés, en ce qui concerne les rapports de leurs bras de levier, de manière qu'une amplification des forces se produise dans le flux des forces depuis la partie de poignée déplaçable (22) jusqu'à l'élément (18 ; 18') de transmission des forces, par l'intermédiaire du dispositif à leviers (82 ; 82').

10. Poignée selon l'une des revendications 1 à 8, **caractérisée en ce que** le premier levier (84 ; 84') et le deuxième levier (86 ; 86') et éventuellement le troisième levier (150) sont réalisés, en ce qui concerne les rapports de leurs bras de levier, de manière que par le dispositif à leviers (82 ; 82') soit transmise une force constante ou moindre.

11. Poignée selon l'une des revendications 1 à 10, **caractérisée en ce que** la tige (12) peut être reliée à la partie d'accouplement (42), de manière à pouvoir tourner autour de son axe longitudinal (66).

12. Poignée selon l'une des revendications 1 à 11, **caractérisée en ce que** l'élément (18) de transmission des forces peut être relié directement ou indirectement au deuxième levier (86), par l'intermédiaire d'une liaison à pivot sphérique et coussinet sphérique.

13. Poignée selon l'une des revendications 1 à 12, **caractérisée en ce que** la partie d'accouplement (42) peut être bloquée au moyen d'un mécanisme à cran d'arrêt (70), dans plusieurs positions angulaires par rapport à l'axe de poignée (68), dans une plage angulaire totale pouvant aller jusqu'à environ 240°, autour du premier axe de rotation (64) et/ou autour du deuxième axe de rotation (138).

14. Poignée selon la revendication 13, **caractérisée en ce que** le mécanisme à cran d'arrêt (70) comporte un ergot d'arrêt (76) qui peut être amené en prise avec des découpes (72) de la partie d'accouplement (42), le mécanisme à cran d'arrêt (70) comportant en outre un bouton-pression (74), de sorte que par enfoncement du bouton-pression (74), l'ergot d'arrêt (76) peut être dégagé de la découpe (72) correspondante.

15. Poignée selon l'une des revendications 1 à 14, **caractérisée en ce que** la partie d'accouplement (42) comporte un logement pour la tige (12), en vue de la liaison amovible de la tige (12) avec la partie d'accouplement (42).

16. Poignée selon l'une des revendications 1 à 15, **caractérisée en ce que** la partie d'accouplement (42) comporte un logement (50) pour une extrémité proximale de l'élément (18) de transmission des forces, le logement (50) étant déplaçable axialement et relié au deuxième levier (86).

17. Poignée selon la revendication 16, **caractérisée en ce que** le logement (50) comporte un mécanisme à cran d'arrêt (52) pour la liaison amovible de l'élément (18) de transmission des forces avec la partie d'accouplement (42).

18. Instrument médical comportant une tige (12), comportant au moins un outil déplaçable (16) à l'extrémité distale de la tige (12), comportant un élément (18 ; 18') de transmission des forces, disposé déplaçable axialement dans la tige (12) pour actionner le ou les outil(s) déplaçable(s) (16), **caractérisé par** une poignée (20 ; 20') selon l'une des revendications 1 à 17.

19. Instrument selon la revendication 18, **caractérisé en ce que** la tige (12) est reliée de manière amovible à la poignée (20 ; 20').

20. Instrument selon la revendication 18 ou 19, **caractérisé en ce que** l'élément (18; 18') de transmission des forces est relié de manière non permanente à la poignée (20 ; 20').

21. Instrument selon l'une des revendications 18 à 20, **caractérisé en ce que** l'élément (18 ; 18') de transmission des forces est relié de manière amovible à la tige (12).
